# EUROPEAN PATENT APPLICATION

(11) **EP 4 368 177 A1**
(43) Date of publication of application: **15.05.2024**
(21) Application number: 22853381.6
(22) Date of filing: 01.08.2022
(51) Int. Cl.: A61K 31/165, A61P 17/02, A61P 43/00

(54) **COMPOSITION FOR TREATING HYPERTROPHIC SCARS AND KELOIDS COMPRISING BENZHYDRYL THIOACETAMIDE COMPOUND AS ACTIVE INGREDIENT**

(30) Priority: 02.08.2021 KR 20210101359; 29.07.2022 KR 20220094406
(71) Applicant: Cellionbiomed Inc., Daejeon 34013 (KR)
(72) Inventor: KIM, Seong-Jin, Sejong 30062 (KR); KIM, Seon-Myung, Daejeon 34018 (KR)
(74) Representative: BCKIP Part mbB
(86) International application number: PCT/KR2022/011265
(87) International publication number: WO 2023/014003

(57) **Abstract**

The present invention relates to a composition for treating hypertrophic scars and keloids, comprising a benzhydryl thioacetamide compound as an active ingredient. The benzhydryl thioacetamide compound inhibits cell proliferation and an increase in intracellular Ca²⁺ concentration, in fibroblasts, keratinocytes and vascular endothelial cells that are involved in skin fibrosis, and inhibits the synthesis of collagen, which is the extracellular matrix, and thus has the effect of effectively treating intractable hypertrophic scars and keloids.

## Description

### [Technical Field]

The present invention relates to a composition for treating hypertrophic scars and keloids, which includes a benzhydryl thioacetamide compound as an active ingredient, and more particularly, to a composition for treating hypertrophic scars and keloids, which includes a benzhydryl thioacetamide compound, wherein the benzhydryl thioacetamide compound suppresses an intracellular increase in Ca²⁺ concentration and cell proliferation in fibroblasts, keratinocytes, and vascular endothelial cells, which are involved in skin fibrosis, and suppresses the synthesis of collagen, such as the extracellular matrix (ECM), exhibiting the efficacy of effectively treating intractable hypertrophic scars and keloids.

### [Background Art]

Generally, the process of healing wounds in the human body includes three steps: inflammation, proliferation, and reconstruction. First, in the inflammation step, the wound area is hemostatic, and immune cells including white blood cells and neutrophils eliminate harmful materials entering from the outside and damaged tissue. Next, in the proliferation step, vascular endothelial cells migrate to the wound area to form new blood vessels in the damaged tissue, extracellular tissue-forming proteins such as collagen and elastin are produced, and fibroblasts form granulation tissue.

Finally, in the reconstruction step, as the proliferated epithelial cells become mature, and fibrous tissue is formed, the adhesive force and elasticity between newly synthesized tissue and proliferated cells increase. Such a wound healing process may take several months to years depending on the severity of the wound. In addition, when the skin is damaged deep into the dermal layer, collagen proliferates in the dermal layer that maintains skin tension, resulting in leaving `scars,' which are healing marks on the skin.

When fibrous tissue becomes excessively dense during wound healing in the human body, a `hypertrophic scar' or `keloid' in which the wound area grows abnormally enlarged may be formed. A hypertrophic scar (also called an `enlarged scar') is no larger than the wound area and tends to gradually disappear over time, whereas a keloid grows larger than the wound area over time, invading the normal skin.

A hypertrophic scar or a keloid, which is a type of skin fibrosis, occurs when cell migration is excessively activated during the wound healing process, or the cells and capillaries of tissue proliferate abnormally, and collagen accumulates excessively. Statistically, hypertrophic scars or keloids are very common, appearing within one year in 40 to 70% of surgical patients, but a therapeutic agent that can effectively suppress this condition has not yet been developed.

Previously, efforts have been made to develop therapeutic agents that can effectively suppress a hypertrophic scar or keloid, or skin fibrosis. For example, in Korean Patent No. 10-2026138 (published on September 23, 2019), a pharmaceutical composition for suppressing or treating keloid and hypertrophic scar formation, including the expression inhibitor for CR6 interacting factor 1 (CRIF1) gene as an active ingredient, is suggested. CRIF1 is a type of protein present in the mitochondria, and includes, as its active ingredient, an antisense nucleotide or siRNA, which specifically binds to the CRIF1 gene.

In addition, in Korean Patent No. 10-2206017 (published on January 15, 2021), a pharmaceutical composition for preventing or treating a skin fibrosis disease, which includes an expression or activity inhibitor of poly ADP-ribose polymerase 1 (PARP1) as an active ingredient, is disclosed. As the active ingredient, rucaparib, which is used as a therapeutic agent for ovarian cancer, is exemplified.

In Korean Patent No. 10-2232072 (published on March 19, 2021), a composition for preventing or treating skin fibrosis, which includes a compound represented by Formula A below or a pharmaceutically acceptable salt thereof as an active ingredient, is suggested. 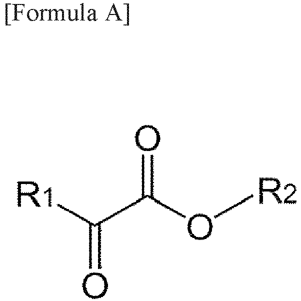 (In Formula A, R₁ and R₂ are each independently C1 to C3 alkyl.)

The compound of Formula A inhibits the expression of high-mobility group box 1 (HMGB1), collagen I, collagen III, fibronectin, and elastin in fibroblasts. In addition, the representative compound among the compounds of Formula A is ethyl pyruvate modified from pyruvic acid.

Meanwhile, in Korean Patent No. 10-1947277 (published on February 01, 2019), a pharmaceutical composition for preventing or treating hypertrophic scar formation, which contains the K_{Ca}3.1 channel blocker 1-[(2-chlorophenyl)diphenylmethyl]-1H-pyrazole (TRAM-34), is disclosed. The K_{Ca}3.1 channel is a type of potassium (K⁺) channel specifically distributed in fibroblasts and is mainly involved in the activity of fibroblasts.

In addition, Korean Patent No. 10-1414831 (published on June 26, 2014), it has been reported that modafinil, known as a therapeutic agent for narcolepsy, and derivatives thereof are effective on inhibiting the current of the K_{Ca}3.1 channel, and can be used as a therapeutic agent for K_{Ca}3.1 channel-mediated diseases, such as sickle cell anemia, autoimmune diseases, cancer diseases, traumatic brain injury, neurodegenerative diseases, and secretory diarrhea.

In addition, in Korean Patent No. 10-2277739 (published on July 09, 2021) and the corresponding International Publication No. WO2020/055166A1 (published on March 19, 2020), a composition for treating a liver fibrosis or pulmonary fibrosis disease, which includes a benzhydryl thioacetamide compound as an active ingredient, has been disclosed. The benzhydryl thioacetamide compound includes modafinil and is effective in inhibiting the expression of the K_{Ca}2.3 channel protein in the cell membrane.

### [Disclosure]

### [Technical Problem]

When most cells are stimulated, a Ca²⁺ concentration in the cells increases and their function is activated. Ca²⁺ acts as a secondary messenger in an intracellular signaling process and plays a very important role in regulating cell function. Even in a skin fibrosis process, intracellular Ca²⁺ plays a very important role in activating fibrosis-related cells, forming myofibroblasts, and regulating the formation and secretion of extracellular matrix. Therefore, when the increase in intracellular Ca²⁺ concentration is inhibited, the formation of skin fibrosis, particularly, the formation of hypertrophic scars and keloids, may be effectively inhibited.

Accordingly, the present invention is directed to providing a novel pharmaceutical composition that inhibits the increase in intracellular Ca²⁺ concentration cell proliferation in skin fibrosis-related cells, and further inhibits the synthesis of collagen, which is extracellular matrix, resulting in effectively treating the formation of hypertrophic scars and keloids.

The present invention is also directed to providing a novel pharmaceutical composition that can increase therapeutic efficacy on hypertrophic scars and keloids by coadministration with a conventionally developed therapeutic agent for hypertrophic scars, such as TRAM-34 that is effective in blocking the K_{Ca}3.1 channel.

For reference, the meaning of the term "treatment" used herein includes inhibiting the development of a condition, a disease, or a symptom, and alleviating or eliminating the same.

### [Technical Solution]

A composition for treating hypertrophic scars and keloids according to the present invention includes a benzhydryl thioacetamide compound represented by Formula 1 below or a pharmaceutically acceptable salt thereof as an active ingredient. 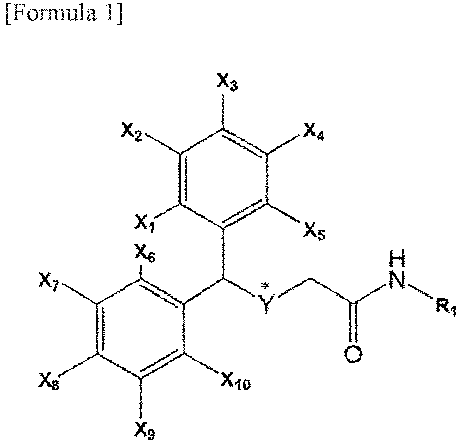 [In Formula 1, X₁ to X₁₀ are any of hydrogen (H), fluorine (F), and chlorine (Cl), and may be the same as or different from each other; Y is sulfur (S) or sulfoxide (S=O), * indicates a chiral body; and Ri indicates any one of hydrogen, a hydroxyl group, a methyl group, an ethyl group, a propyl group, an isopropyl group, a vinyl group, an acryl group, a butyl group, a t-butyl group, an isobutyl group, a furan group, a hydrofuran group, a cyclopropane group, an oxirane group, and a cyclic phenyl group.]

The benzhydryl thioacetamide compound represented by Formula 1 is effective in inhibiting the increase in Ca²⁺ concentration and cell proliferation in fibroblasts, keratinocytes, and vascular endothelial cells, and inhibiting the synthesis of collagen, which is extracellular matrix.

### [Advantageous Effects]

A benzhydryl thioacetamide compound according to the present invention, represented by Formula 1, can effectively treat intractable hypertrophic scars and keloids. It can be coadministered with a conventional hypertrophic scar therapeutic agent that is effective in inhibiting a potassium channel as needed, and is expected to be used as a composition for a therapeutic adjuvant or cosmetic.

### [Description of Drawings]

FIG. 1 is a set of graphs assessing the inhibitory effects of CBM-N1 to N10 compounds according to the present invention on intracellular Ca²⁺ increase in fibroblasts.
FIG. 2 is a set of graphs assessing the inhibitory effects of CBM-N1, N3, and N5 compounds according to the present invention on intracellular Ca²⁺ increase in keratinocytes and vascular endothelial cells.
FIG. 3 is a set of graphs assessing the inhibitory effects of La³⁺, which is a material for blocking intracellular Ca²⁺ influx, on intracellular Ca²⁺ increase in fibroblasts.
FIG. 4 is a set of graphs assessing the inhibitory effects of CBM-N1 to N5, and N8 to N10 compounds according to the present invention on cell proliferation in fibroblasts.
FIG. 5 is a set of graphs assessing the inhibitory effects of CBM-N1 to N10 compounds and La³⁺ according to the present invention on cell proliferation in keratinocytes.
FIG. 6 is a set of graphs assessing the inhibitory effects of CBM-N1 to N8 compounds according to the present invention on fibrosis in fibroblasts.
FIG. 7 is a set of graphs assessing the inhibitory effects of CBM-N1 to N7 compounds according to the present invention on fibrosis in fibroblasts.
FIG. 8 is sets of images and graphs, which analyze the effects of CBM-N1, N3, N5, and N9 compounds by observing hypertrophic scars by a skin magnifier in hypertrophic scar models using rabbit ears.
FIG. 9 is a set of microscopic images and a graph, which analyze the effects of CBM-N1, N3, N5, and N9 compounds by measuring the epidermal thicknesses of hypertrophic scars in hypertrophic scar models using rabbit ears.
FIG. 10 is a set of microscopic images and a graph, which analyze the effects of CBM-N1, N3, N5, and N9 compounds by measuring scar elevation indexes (SEIs) in hypertrophic scar models using rabbit ears.
FIG. 11 is a set of microscopic images and a graph, which analyze the effects of CBM-N1, N3, N5, and N9 compounds by measuring collagen densities in hypertrophic scar models using rabbit ears.

### [Modes of the Invention]

Hereinafter, the present invention will be described in detail with reference to the accompanying drawings. However, even if the configuration is essential for carrying out the present invention, detailed description will be omitted for matters that were introduced in the prior art or that can be easily implemented by one skilled in the art from known techniques.

The benzhydryl thioacetamide compound according to the present invention, represented by Formula 1, includes, particularly, CBM-N1 to CBM-N10 compounds below. Here, CBM-N1 to N10 are code names given by the present inventors, the chemical names of which are as follows.
1) CBM-N1 ; 2-(benzhydrylsulfinyl)acetamide
2) CBM-N2 ; 2-(benzhydrylthio)-N-[(tetrahydrofuran-2-yl)methyl]acetamide
3) CBM-N3 ; 2-(benzhydrylthio)-N-phenylacetamide
4) CBM-N4 ; 2-(benzhydrylsulfinyl)-N-methylacetamide
5) CBM-N5 ; 2-(benzhydrylsulfinyl)-N-[(tetrahydrofuran-2-yl)methyl]acetamide
6) CBM-N6 ; 2-(benzhydrylthio)-ene-methylacetamide
7) CBM-N7 ; 2-[bis(2-fluorophenyl)methanesulfinyl]acetamide
8) CBM-N8 ; 2-[bis(3-fluorophenyl)methanesulfinyl]acetamide
9) CBM-N9 ; 2-[bis(4-fluorophenyl)methanesulfinyl]acetamide
10) CBM-N10 ; 2-[bis(4-chlorophenyl)methanesulfinyl]acetamide

The CBM-N1 compound is known by the common name 'modafinil,' and the CBM-N9 compound is known by the common name `lauflumide.' In addition, all the CBM-N1 to N10 compounds show almost the same pharmacological mechanisms in the human body.

The CBM-N1 compound may be prepared by a variety of known methods, or commercially available ingredients may be purchased and used. In addition, methods of preparing the CBM-N2 to N6 compounds are known from Korean Patent No. 10-1345860, and methods of preparing the CBM-N7 to N9 compounds are known from International Publication No. WO 2020/ 055166A1.

The CBM-10 compound, as a novel compound, may be prepared by the same method as the CBM-9 compound. However, as a starting material, bis(4-chlorophenyl)methanol is used instead of bis(4-fluorophenyl)methanol. The ¹H NMR (DMSO-d6) of the CBM-10 compound prepared by the above method was analyzed to be 7.3 to 7.7 ppm (m, 8H), 5.4 ppm (s, 1H), 3.45 ppm (d, 1H), and 3.2 ppm (d, 1H).

Other than the CBM-N1 to N10 compounds, 2-(benzhydrylsulfinyl)-N-hydroxyacetamide known by the common name 'adrafinil' is as a precursor of the CBM-N1 compound, i.e., modafinil, and has the same pharmacological action as modafinil (CNS Drug Reviews Vol5, No.3 193-212, 1999).

The compound of Formula 1 inhibits the increase in intracellular Ca²⁺ concentration in fibroblasts, keratinocytes, or vascular endothelial cells, which are involved in skin fibrosis. Intracellular Ca²⁺ is involved in the activation of fibrosis-related cells, the formation of myofibroblasts, and the formation and secretion of extracellular matrix, promoting the formation of hypertrophic scars or keloids.

In addition, the compound of Formula 1 inhibits the function of keratinocytes, which play an auxiliary role in the skin fibrosis process. Keratinocytes allow various types of growth factors to be secreted and stimulate fibroblasts to produce growth factors. In addition, keratinocytes stimulate the formation of myofibroblasts and thus contribute to the formation of hypertrophic scars and keloids.

In addition, the compound of Formula 1 inhibits the function of vascular endothelial cells that play an auxiliary role in the skin fibrosis process. Vascular endothelial cells contribute to the growth of fibroblast tissue by generating new blood vessels and facilitating the blood supply necessary for the growth of fibroblast tissue.

The pharmaceutical composition according to the present invention includes a pharmaceutically acceptable salt of the compound of Formula 1. Here, the "pharmaceutically acceptable salt" encompasses common metal salts, salts with organic bases, salts with inorganic acids, and salts with basic or acidic amino acids.

The pharmaceutical composition according to the present invention includes both a solvate and a hydrate of the compound of Formula 1 and may also include all possible stereoisomers. The solvate, hydrate, and stereoisomer may be prepared using conventional methods. In addition, the pharmaceutical composition according to the present invention may include a crystalline or amorphous form of the compound of Formula 1, and when prepared in a crystalline form, may optionally be hydrated or solvated.

The pharmaceutical composition according to the present invention may be prepared in various formulations alone or in combination with a pharmaceutically acceptable carrier, excipient (forming agent) or diluent by a method known in the pharmaceutical field. For example, the pharmaceutical composition according to the present invention may be prepared in oral formulations such as powder, granules, tablets, capsules, syrups, and emulsions, formulations for external use, or injections.

Particularly, considering that the use of the composition according to the present invention is treatment for hypertrophic scars and keloids formed by skin wounds, the pharmaceutical composition according to the present invention is preferably formed in an external preparation for skin or injection, which can be administered topically. For the external preparations for skin, liquids, ointments, creams, lotions, sprays, patches, gels, or aerosols may be used.

The composition according to the present invention may be administered in a pharmaceutically effective amount. The effective amount may be determined by considering all factors including the type, size and depth of a wound, a patient's age and sex, drug activity, sensitivity to a drug, administration time, administration route, excretion rate, duration of treatment, and concurrent drugs.

A preferable daily effective dose of the compound of Formula 1 may be 0.2 to 20 mg/kg, and particularly, 0.5 to 10 mg/kg, and administered at once or in two divided portions a day, but the present invention is not limited thereto.

The therapeutic effect of the benzhydryl thioacetamide compound according to the present invention on hypertrophic scars and keloids was tested as below.

### [In vitro tests using fibroblasts, keratinocytes, and vascular endothelial cells]

### 1) Test method

Fibroblasts NIH-3T3 (CRL-2795; American Type Culture Collection, VA, USA) and vascular endothelial cells in the mouse aorta were cultured in Dulbecco's Modified Eagle Medium (Hyclone, Logan, UT), and keratinocytes (HaCaT keratinocyte) were cultured in MV2 medium (PromoCell GmbH).

The fibroblasts were exposed to the fibrosis-causing material TGF-β (5 ng/mL), and the keratinocytes to ATP (10 µM) to increase intracellular Ca²⁺ concentrations, that is [Ca²⁺]ᵢ.

When [Ca²⁺]ᵢ reached a steady state, each of the CBM-N1 to N10 compounds was administered to observe its effect on [Ca²⁺]ᵢ.

Meanwhile, a method of measuring [Ca²⁺]ᵢ is as follows. First, the cells were cultured on a cover glass in a cell culture plate (12 wells), and a Ca²⁺ sensitive dye Fura-2/AM (2 µM) was added to the culture medium to penetrate the cells at 37 °C for 25 minutes.

360 and 380 nm light sources were alternately applied at 50 Hz to the cells using a photometric Ca²⁺ spectrophotometer (Photon Technology International Inc) of an inverted microscope (DM IRB, Leica, Germany). Fluorescence emitted from the cells was measured at 510 nm, and the intracellular Ca²⁺ concentration was converted from the result. In addition, to block the influx of Ca²⁺ through various Ca²⁺ channels, La³⁺ (10 µM) was used.

After exposing the fibroblasts and the keratinocytes to each of TGF-β (5 ng/mL), TGF-β+CBM-N1 to N10, and TGF-β+La³⁺ for 24 hours, the effect on cell proliferation was analyzed by an MTT assay or CCK-8 assay.

In addition, the fibroblasts were exposed to either TGF-β (5 ng/mL) or TGF-β+CBM-N1 to N7 for 24 hours, and the expression levels of fibrosis markers, such as α-smooth muscle actin (α-SMA), collagen 1α (Col1α), and collagen 3α (Col3α), were analyzed by Western blot or PCR.

### 2) Test results

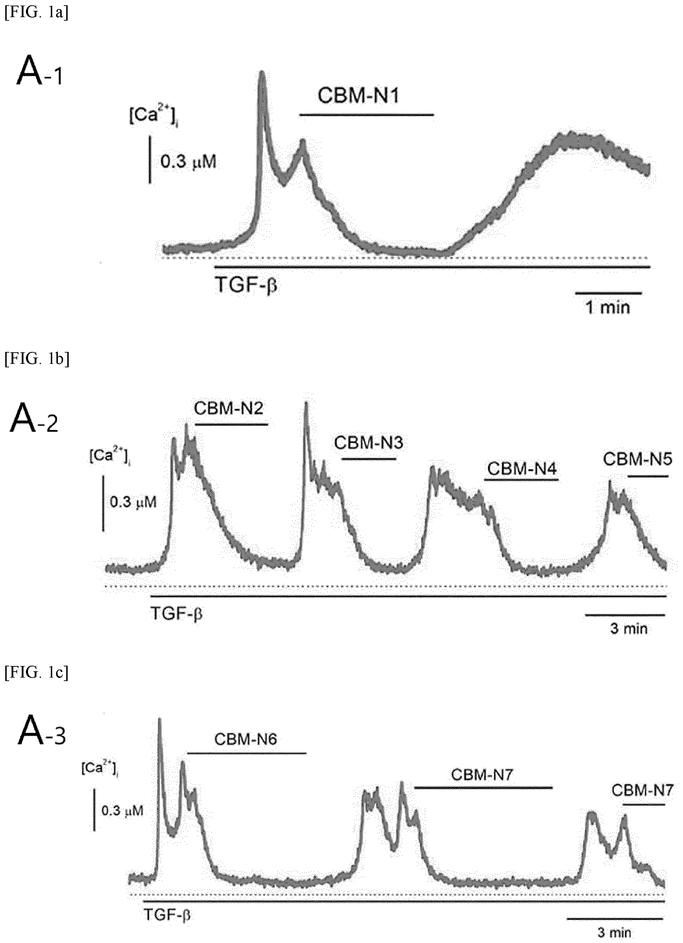

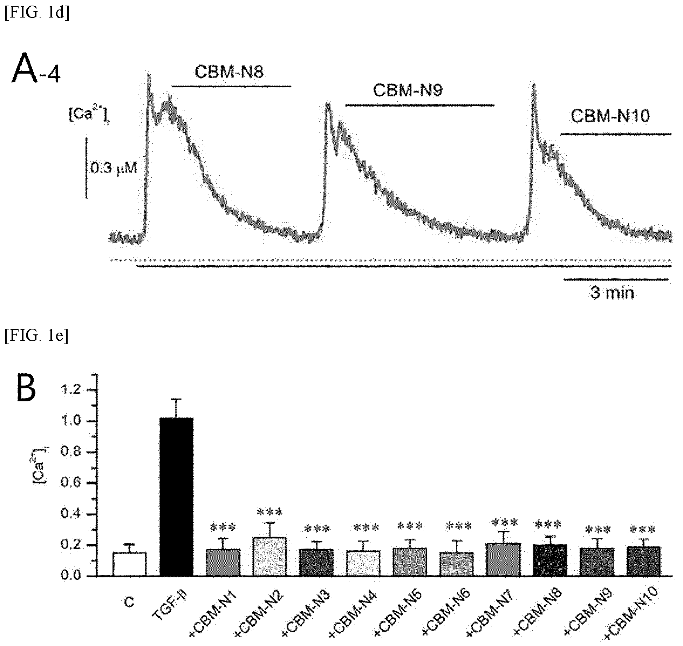

FIG. 1 shows the increase in intracellular Ca²⁺ concentration ([Ca²⁺]ᵢ) from TGF-β (5 ng/mL) and the CBM-N1 to N10 effects thereon in fibroblasts (indicated as mean ± SE, n=5. *** < 0.001).

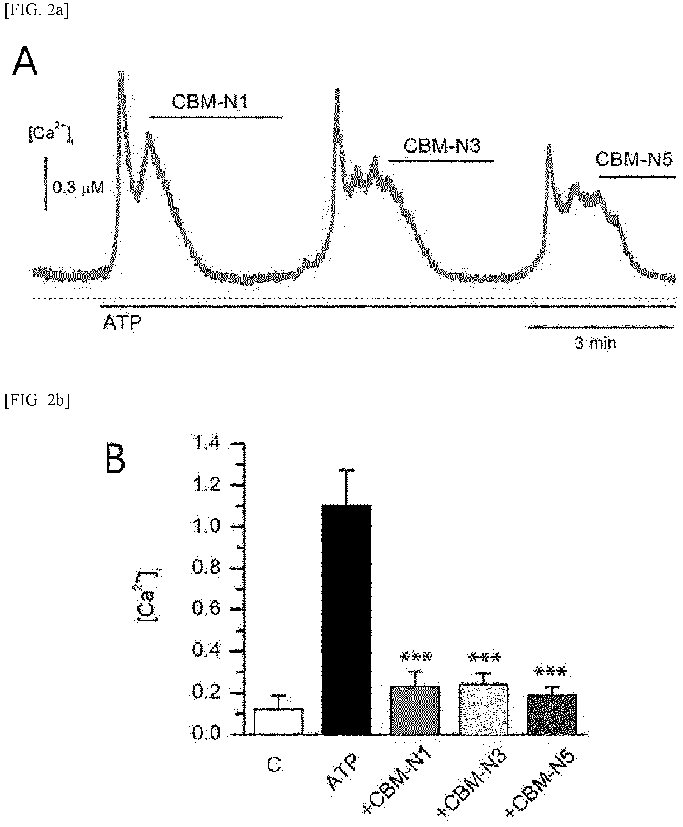

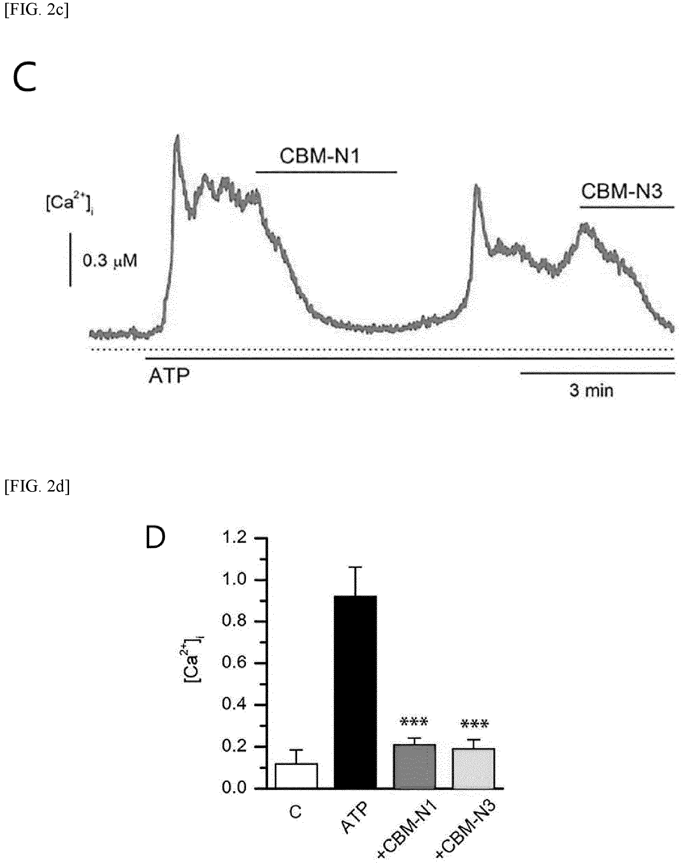

FIG. 2 shows the increase in [Ca²⁺]ᵢ from ATP (10 µM) and CBM-N1, N3, and N5 effects thereon in keratinocytes (A and B) and vascular endothelial cells (C and D) (indicated as mean ± SE, n=5. *** < 0.001).

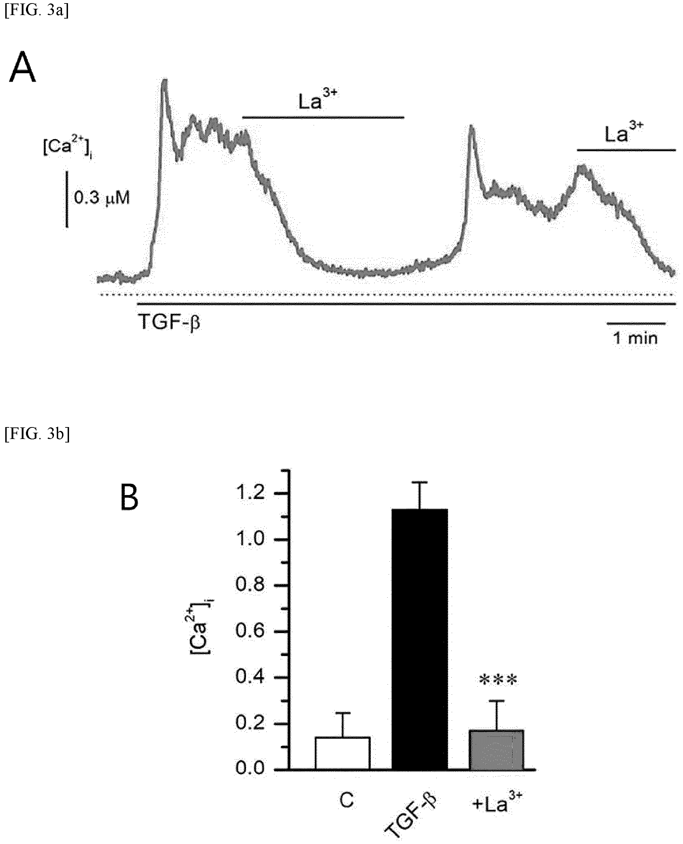

FIG. 3 shows the increase in [Ca²⁺]ᵢ from TGF-β (5 ng/mL) and a La³⁺ (10 µM) effect thereon in fibroblasts (indicated as mean ± SE, n=5. *** < 0.001). The La³⁺ (10 µM) is known to block the intracellular influx of Ca²⁺ through various Ca²⁺ channels.

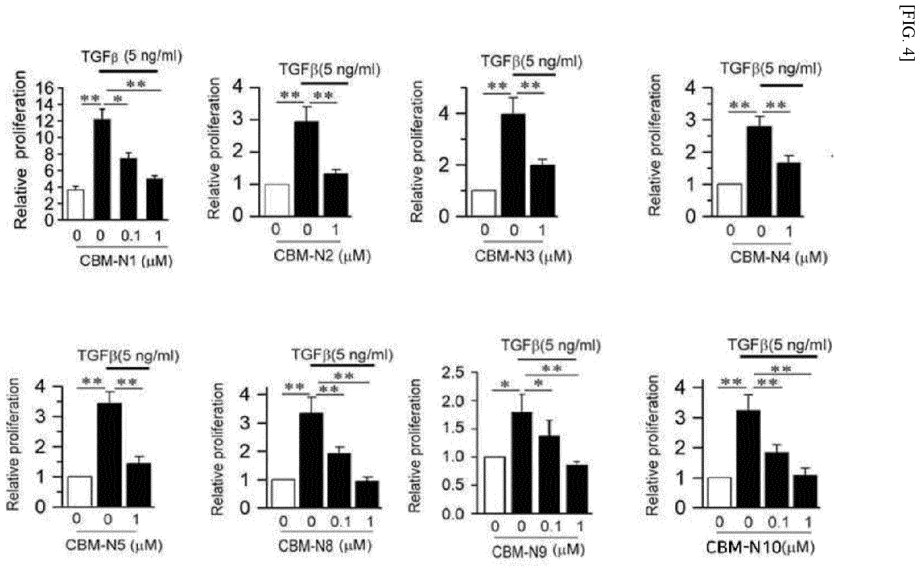

FIG. 4 shows the effects of CBM-N1 to N5 and N8 to N10 compounds in blocking cell proliferation (relative proliferation) from TGF-β (5 ng/mL, exposed for 24 hours) in fibroblasts (indicated as mean ± SE, n=5. * < 0.05, ** < 0.01). The cell proliferation was measured by an MTT assay.

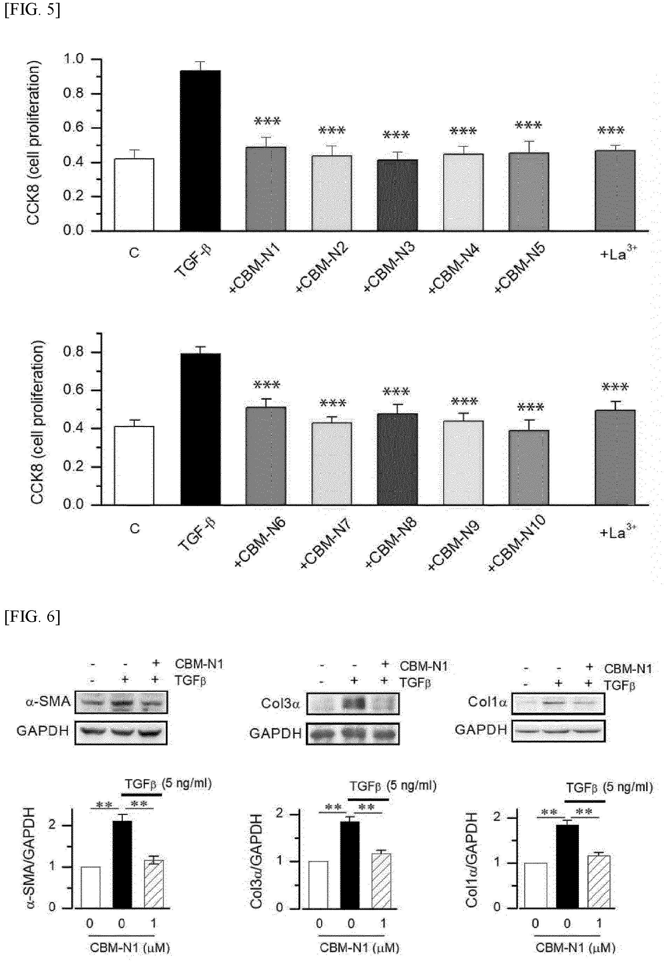

FIG. 5 shows the effects of CBM-N1 to N10 compounds in blocking cell proliferation from TGF_{-β} (5 ng/mL, exposed for 24 hours) in keratinocytes (indicated as mean ± SE, n=5. *** < 0.001). The cell proliferation was measured by a CCK-8 assay.

FIG. 6 shows the effect of CBM-N1 compound (1 µM) on the expression of α-smooth muscle actin (α-SMA), collagen 3α (Col3α), and collagen 1α (Col1α) in fibroblasts exposed to TGF-β (5 ng/mL) for 24 hours (indicated as mean ± SE, n=5. ** < 0.01). The expression of α-SMA, Col3α, and Col1α proteins was measured by a Western blot method.

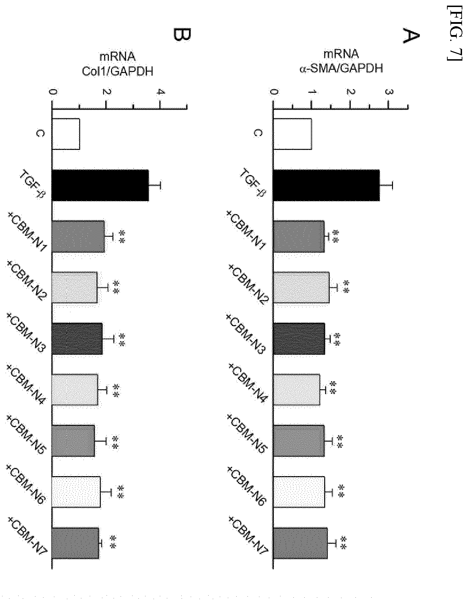

FIG. 7 shows the effects of CBM-N1 to N7 compounds (1 µM) on the expression of α-SMA and Col1α in fibroblasts exposed to TGF-β (5 ng/mL) for 24 hours (indicated as mean ± SE, n=5. ** < 0.01). The expression of α-SMA and Col1α mRNAs was measured by RT-PCR.

### 3) Evaluation and analysis

As shown in FIGS. 1 and 2, and 4 and 5, it was shown that the CBM-N1 to N10 compounds of the present invention effectively inhibit the increase in intracellular Ca²⁺ concentration and cell proliferation by TGF-β or ATP in fibrosis-related cells, such as fibroblasts, keratinocytes, and vascular endothelial cells.

In addition, as shown in FIGS. 3 and 5, when the intracellular influx of Ca²⁺ was blocked using La³⁺, it was confirmed that the increase in cell proliferation was inhibited. These results mean that the CBM-N1 to N10 compounds of the present invention inhibit the intracellular influx of Ca²⁺ to inhibit cell proliferation.

In addition, as shown in FIGS. 6 and 7, the CBM-N1 to N7 compounds inhibited the increase in expression of α-SMA, Col1α, or Col3α by the fibrosis-causing material, TGF-β. The intracellular increase in Ca²⁺ in fibroblasts is known to play an important role in formation of α-SMA and the extracellular matrix, collagen. Accordingly, the inhibition of the expression of α-SMA, Col1α, or Col3α by the CBM-N1 to N7 compounds of the present invention is presumed to be a result of the inhibition of Ca²⁺ increase.

For reference, the most important step in the fibrosis process is the formation of myofibroblasts, and myofibroblasts should be formed for the fibrosis process to occur. Myofibroblasts are formed by the activation of fibroblasts or the epithelial mesenchymal transition of vascular endothelial cells or smooth muscle cells.

α-SMA is a marker of myofibroblasts, and the increase in α-SMA expression indicates that myofibroblasts are generated. In addition, when myofibroblasts are generated, collagen production increases. Accordingly, the increases in α-SMA expression and collagen formation indicate the formation of myofibroblasts, which is a very significant basis for the fibrosis process.

The above test results show that the benzhydryl thioacetamide compound according to the present invention, represented by Formula 1, inhibits the activation of fibroblasts, thereby suppressing skin fibrosis.

### [In vivo test using rabbit hypertrophic scar models]

### 1) Preparation of test models

Eight female New Zealand white rabbits (2.6 to 3 kg) were anesthetized, and wounds were formed by removing the epidermis, dermis, and perichondrium of the ear skin of the rabbits using an 8 mm biopsy punch. Three wounds were formed in an ear area of each rabbit. The rabbit's surgical site was sterilized, and the formation of hypertrophic scars was induced through natural healing for 3 weeks.

After 3-week natural healing, the complete epithelialization and hypertrophic scar formation in scar areas were confirmed, and treatment was started for each test group. The test groups were classified into a normal group (normal tissue), a control (untreated scar/hypertrophic scar), test group 1 (solve application), test group 2 (CBM-N1 10%), test group 3 (CBM-N3 10%), test group 4 (CBM-N 10%), and test group 5 (CBM-N 10%).

Each test group was treated by applying 0.5 mL of drug daily for 2 weeks (14 days), and then a tissue autopsy was performed. The biopsied tissue was stored in a 10% formalin solution to form a tissue slide. The effectiveness of hypertrophic scar treatment was confirmed through dermatoscopy and histological evaluation.

### 2) Visual evaluation

The skin tissue areas of the control and test groups were observed using a dermatoscope on the start day and end day (Day 14 of treatment) of treatment, and depending on the degree of hypertrophic scar formation, scores were given for visual evaluation as shown in Table 1 below.

**[Table 1]**

| Score | Degree of formation of hypertrophic scar |
|---|---|
| 0 | Normal scar |
| 1 | Scar that has imperceptibly low height and is slightly hard |
| 2 | Scar that is slightly swollen and hard |
| 3 | Scar that is swollen and severely hard |

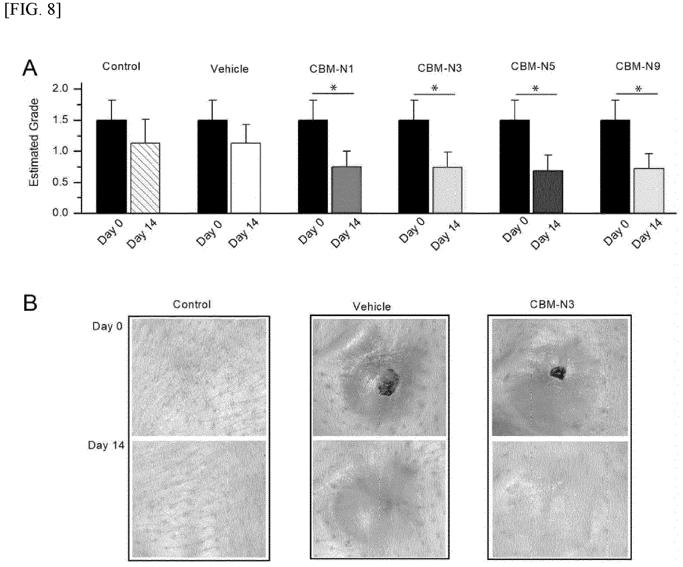

As shown in FIG. 8, there was no significant difference in visual evaluation scores between the control and each test group at the time (day 0) when the hypertrophic scars had formed during natural healing for 3 weeks after cuts. This indicates that hypertrophic scars had formed similarly in all test groups.

However, at the end (day 14) of two-week treatment, compared to the control (Control) and the solvent-treated group (Vehicle), in the test groups treated with CBM-N1, N3, N5, and N9 compounds, the evaluation score of each group was much lower. A lower evaluation score means that the size of a hypertrophic scar has been reduced close to that of normal scars.

### 3) Analysis of epidermis thickness

A paraffin block of the fixed ear tissue of a rabbit was prepared, and sliced to a thickness of 3 µm, thereby preparing a tissue slide. Following hydration, staining was performed using hematoxylin and eosin solutions. The stained tissue slides were photographed using a 200× microscope, and epithelial thicknesses were measured using ZEISS ZEN Microscope Software.

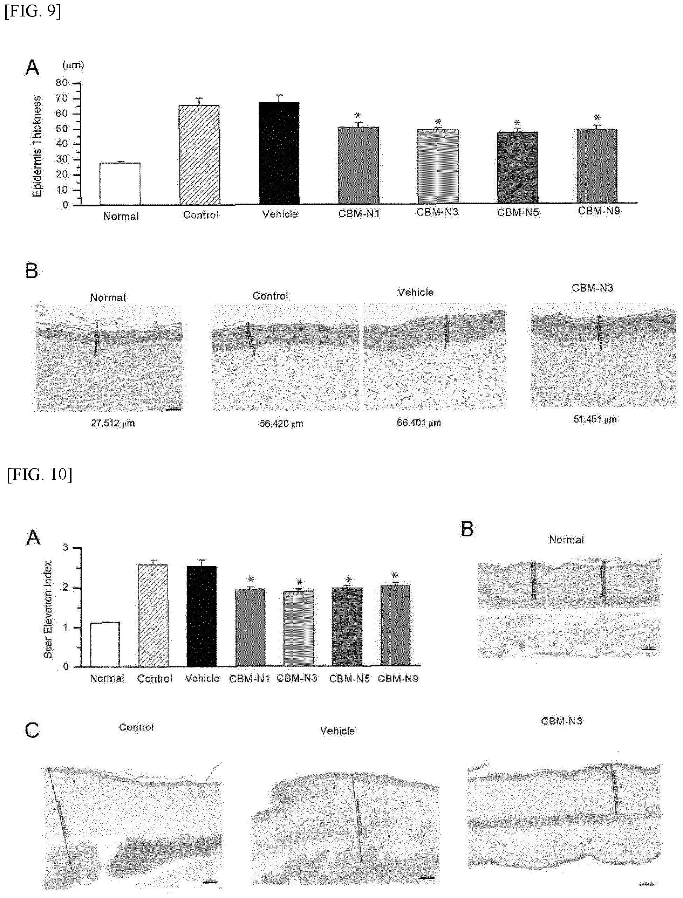

As shown in FIG. 9, in the control (Control) and solvent-treated group (Vehicle) in which hypertrophic scars had formed, compared to the normal tissue (Normal), the epithelial thicknesses increased significantly (p<0.05), and in the CBM-N1, N3, N5, and N9-treated groups, compared to the control and the solvent-treated group, average thin epidermal thicknesses were observed.

### 4) Scar elevation index analysis

The fixed ear tissue of a rabbit was stained with a hematoxylin or eosin solution in the same manner as in the epidermal thickness analysis method, and the stained tissue slide was photographed at 50× magnification. Using ZEISS ZEN Microscope Software, the length from the epidermis to the cartilage was measured for the scar area and normal area, and the scar evaluation index (SEI) was calculated from the results. Here, the SEI is the value calculated by dividing 'the length from the epidermis to the cartilage at the thickest point in the scar area' by 'the length from the epidermis to the cartilage in the normal skin area. '

As shown in FIG. 10, in the control (Control) and solvent-treated group (Vehicle) in which hypertrophic scars had formed, compared to the normal tissue (Normal), the SEI values significantly increased (p<0.05), but in the CBM-N1, N3, N5, and N9 treated groups, compared to the control and the solvent-treated group, lower SEI values were observed on average.

### 5) Collagen density analysis

A paraffin block of the fixed ear tissue of a rabbit was prepared, and sliced to a thickness of 3 µm, thereby preparing a tissue slide. Following hydration, staining was performed using Biebrich Scarlet-Acid Fuchsin solution for 5 minutes, and washing was performed and then staining was performed again with phosphotungstic acid/phosphomolybdic acid for 5 minutes.

Subsequently, staining was performed with aniline blue and an acetic acid solution, and the stained tissue slides were photographed at 400× magnification. In the photographs that were taken, the ratio of the blue-stained area, that is, the area occupied by collagen, to the total area was analyzed using Image J (The National Institutes of Health, USA).

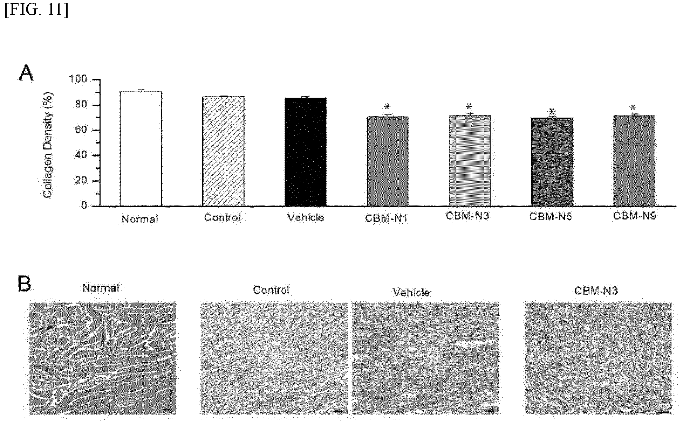

As shown in FIG. 11, in the control (Control) and solvent-treated group (Vehicle) in which hypertrophic scars had formed, compared to the normal tissue (Normal), collagen densities were significantly reduced, and in the CBM-N1, N3, N5, and N9 treated groups, compared to the control and the solvent-treated group, lower collagen densities were observed on average.

### 6) Evaluation and analysis

As shown in FIG. 8, as a result of treating the hypertrophic scars formed by natural healing of the cut in a rabbit ear for 3 weeks with CBM-N1, N3, N5, and N9 compounds, compared to the control or solvent-treated group, the sizes of hypertrophic scars were much smaller. In addition, from the test results of FIGS. 9, 10, and 11, in the test groups treated with CBM-N1, N3, N5, and N9 compounds, epidermal thicknesses, SEI values, and collagen densities were relatively reduced.

These test results show that the CBM-N1, N3, N5, and N9 compounds of the present invention decreased collagen density and epidermal thickness, inhibiting the formation of hypertrophic scars.

### [Conclusion]

It was confirmed that the CBM-N1 to N10 compounds representing the compound of Formula 1 of the present invention inhibit the increase in intracellular Ca²⁺ concentration to similar levels in all the fibrosis-related cells, such as fibroblasts, keratinocytes, and vascular endothelial cells, and further inhibit cell proliferation and the synthesis of α-SMA and the extracellular matrix, collagen. In addition, it was confirmed that the CBM-N1, N3, N5, and N9 compounds can inhibit the formation of hypertrophic scars caused by cuts in rabbit hypertrophic scar models.

Due to realistic conditions, there are limitations in conducting the same efficacy test for all compounds of Formula 1, but according to pharmacokinetic experience, it is expected that other compounds of Formula 1 that are not included in the above tests will exhibit the same or similar pharmacokinetic actions to the CBM-N1 to N10 compounds. Therefore, it can be concluded that all the benzhydryl thioacetamide compounds of Formula 1 according to the present invention have the efficacy of effectively inhibiting or treating the formation of hypertrophic scars and keloids.

## Claims

1. A composition for treating hypertrophic scars and keloids, comprising:
a benzhydryl thioacetamide compound represented by Formula 1 or a pharmaceutically acceptable salt thereof as an active ingredient: 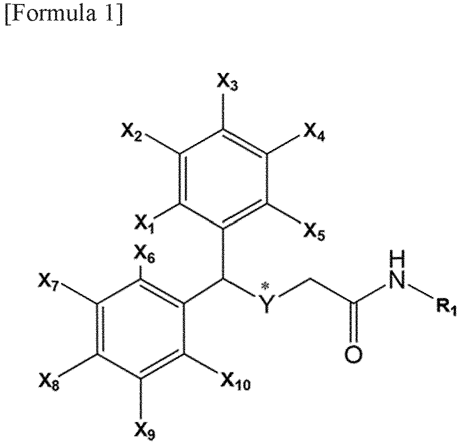 [In Formula 1, X₁ to X₁₀ are any of hydrogen (H), fluorine (F), and chlorine (Cl), and may be the same as or different from each other; Y is sulfur (S) or sulfoxide (S=O), * indicates a chiral body; and Ri indicates any one of hydrogen, a hydroxyl group, a methyl group, an ethyl group, a propyl group, an isopropyl group, a vinyl group, an acryl group, a butyl group, a t-butyl group, an isobutyl group, a furan group, a hydrofuran group, a cyclopropane group, an oxirane group, and a cyclic phenyl group.]

2. The composition of claim 1, wherein the compound of Formula 1 inhibits the increase in Ca²⁺ concentration and cell proliferation in fibroblasts, keratinocytes, and vascular endothelial cells, and inhibits the synthesis of collagen, which is extracellular matrix.

3. The composition of claim 1, wherein the compound of Formula 1 is any one selected from 2-(benzhydrylsulfinyl)acetamide; 2-(benzhydrylthio)-N-[(tetrahydrofuran-2-yl)methyl]acetamide; 2-(benzhydrylthio)-N-phenylacetamide; 2-(benzhydrylsulfinyl)-N-methylacetamide; 2-(benzhydrylsulfinyl)-N-[(tetrahydrofuran-2-yl)methyl]acetamide; 2-(benzhydrylthio)-ene-methylacetamide; 2-[bis(2-fluorophenyl)methanesulfinyl]acetamide; 2-[bis(3-fluorophenyl)methanesulfinyl]acetamide; 2-[bis(4-fluorophenyl)methanesulfinyl]acetamide; 2-[bis(4-chlorophenyl)methanesulfinyl]acetamide; and 2-(benzhydrylsulfinyl)-N-hydroxyacetamide.

4. The composition of claim 1, wherein the compound of Formula 1 is 2-(benzhydrylsulfinyl)acetamide (modafinil).

5. The composition of claim 1, wherein the compound of Formula 1 is 2-(benzhydrylsulfinyl)-N-hydroxyacetamide (adrafinil).

6. The composition of claim 1, wherein the compound of Formula 1 is 2-[bis(4-fluorophenyl)methanesulfinyl]acetamide (lauflumide).

7. The composition of claim 1, wherein the compound of Formula 1 is 2-[bis(4-chlorophenyl)methanesulfinyl]acetamide.
